# EUROPEAN PATENT APPLICATION

(11) **EP 4 722 705 A1**
(43) Date of publication of application: **08.04.2026**
(21) Application number: 24204535.9
(22) Date of filing: 03.10.2024
(51) Int. Cl.: G01N 27/416, B01L 3/00, G01N 33/493, G01N 27/27, G01N 27/30

(54) **DIPSTICK ELECTROCHEMICAL SENSING DEVICE AND CORRESPONDING METHOD FOR THE ASSESSMENT OF AN ANALYTE IN A FLUID SAMPLE**

(71) Applicant: CSEM Centre Suisse d'Electronique et de Microtechnique SA - Recherche et Développement, 2002 Neuchâtel (CH)
(72) Inventor: MIGLIORELLI, Davide, 7203 Trimmis (CH); BURR, Loic, 7242 Luzein (CH); GLASER, Nicolas, 4125 Riehen (CH)
(74) Representative: e-Patent SA

(57) **Abstract**

A dipstick electrochemical sensing device (1) is disclosed, for assessing an analyte in a fluid sample (44), comprising a main body (2) provided with a reference electrode (4) and a working electrode (6, 8, 10),
wherein the reference electrode (4) is located in a reference chamber (18) provided with a first port (20), while the working electrode (6, 8, 10) is located in a measurement chamber (22) provided with a second port (24),
wherein the sensing device (1) is further provided with a bridge (32), connecting the reference and measurement chambers (18, 22) through corresponding interfaces (28, 30), and through which a fluid may flow, at least one interface (30) having fluid flow restricting characteristics which are pressure dependent, and
wherein the measurement chamber (22) is partially delimited by a removable wall (34) so that it can be removed to open a main opening through which the fluid sample can enter inside the measurement chamber (22) and contact the working electrode (6, 8, 10) for the purpose of conducting an assessment.

## Description

### Technical Field

The invention is in the field of the electrochemical assessment of the concentration of at least one specific analyte in a solution, more preferably involving sensing devices capable of conducting such an assessment in the context of single point measurements in fluids, more particularly in liquids, including complex solution matrixes such as biofluids (i.e. blood, urine, saliva, interstitial fluids, etc.).

As far as the present invention is concerned, it relates to a dipstick electrochemical sensing device, for assessing at least one analyte in a fluid sample, comprising a main body provided with at least one reference electrode and at least one working electrode, the reference and working electrodes being connected to corresponding electrical connection pads. The present invention further relates to a method for conducting the assessment of at least one analyte in a fluid sample by implementation of such a sensing device.

### State of the art

Cartridge-shape electrochemical sensing devices, with compact sizes, have been known for some time for conducting assessments in the context of Point-of-Care Testing.

Because of its ease of fabrication and a wide compatibility spectrum, in terms of assessed analytes, Ag/AgCI is the most commonly used material for miniaturising and integrating a reference electrode in a cartridge. However, most of the Ag/AgCI reference electrodes lack a proper liquid junction, exposing the reference electrode to the fluid sample to be assessed and causing a variation in the electrode potential, which has a significant impact on measurement accuracy. A main issue with this approach is that the reference electrode is in direct contact with the solution under test causing variability in the voltage potential recorded at the reference electrode.

One approach to stabilise the reference electrode is the integration of a salt bridge providing an conductive connection between the reference electrode and the working electrode by enabling the flow of ions to maintain electrical neutrality. Such a salt bridge typically contains an inert electrolyte solution (such as KCI) in a channel that could contain diffusion limiting elements like a porous barrier at each end of the bridge that allows ions to pass through, or a membrane or hydrogel all inside the bridge.

The salt bridge has one end in one half-cell of the electrochemical cell and the other end in the other half-cell, which allows ions to move between the two half-cells and helps in maintaining a constant electric potential.

According to particular embodiments, cartridge sensing devices may be incorporated with a sample dilution feature for the controlled metered dilution of biological samples (e.g. blood, plasma, serum, urine, interstitial fluid or cerebrospinal fluid) and of analytes in the diluted samples. An example of such a cartridge sensing device is disclosed in publication WO 2012/075251 A1.

Apart from these cartridges, printed electrochemical sensors are widely used to assess the concentration of a specific analyte in a solution. These sensors are made by printing different types of inks (carbon, silver, gold, platinum) on an insulating or insulated substrate. These inks may be printed as one or several stacked layers, and these sensors might also include an additional functionalization layer on top of the ink layer(s). The composition of the top layer determines the selectivity and sensitivity of the electrodes, allowing analysts to design optimal devices for specific purposes. The development of these sensors has been driven by the need to reduce device size and production costs, and to decrease the sample volume required for each experiment. One of their main advantages is the ability to modify the composition of the implemented inks by adding different metals, enzymes, complexing agents, polymers, etc., which is useful for a multitude of electrochemical analyses.

Thanks to their compact structures and shapes, some of these printed sensing devices can be used in a dipstick like measurement approach, consisting in the direct immersion of the sensing device in the fluid sample to be assessed, in order to immerse the corresponding working electrode therein.

An example of such a dipstick sensing device is for instance disclosed in the publication "Dipstick Sensor Based on Molecularly Imprinted Polymer-Coated Screen-Printed Electrodes for the Single-Shot Detection of Glucose in Urine Samples-From Fundamental Study toward Point-of-Care Application", by Caldara, M. et al., in Advanced Materials Interfaces 2023, 10, 2300182.

However, printed electrochemical sensing devices generally require calibration at multiple analyte concentrations to compensate for electrode variability which, for instance, might be due to degradation over time and/or after being in contact with different fluids, among other factors, and they are designed for the assessment of a single analyte while they do not support an easy way to implement a calibration operation, protect the reference electrode or dilute the sample (if needed).

Thus, it appears that there is a need for an electrochemical sensing device of simple construction and use, which could offer the ease of use of a dipstick-like sensing device, while ensuring a good stability of the reference electrode by preventing possible interferences with the fluid sample to be assessed and, preferably, allowing a single calibration step. More preferably, such a sensing device should further be able to be used to work in dilution condition when needed and to precisely quantify the dilution ratio.

### Disclosure of the invention

An aim of the present invention is to propose a dipstick-like electromechanical sensing device fulfilling the above-mentioned requirements, as well as to allow the implementation of a corresponding measuring method which is precise, reliable and as simple as possible in terms of number and complexity of the steps it comprises.

This aim is achieved by providing a sensing device as mentioned above,
wherein the reference electrode is located in a first cavity, defining at least part of a reference chamber and provided with a first inlet and/or outlet port, while the working electrode is located in a second cavity, defining at least part of a measurement chamber and provided with a second inlet and/or outlet port,
wherein the sensing device is further provided with a third cavity connected to both reference and measurement chambers through corresponding interfaces, to define a bridge therebetween through which a fluid may flow, at least the interface located between the bridge and the measurement chamber having such a structure that it is provided with fluid flow restricting characteristics which are pressure dependent, and
wherein the measurement chamber is partially delimited by a removable wall which is assembled to the main body in a removable manner, so that it can be removed to open a main opening through which the fluid sample can enter inside the measurement chamber and contact the at least one working electrode for the purpose of conducting an assessment.

These features of the present invention allow the provision of a reliable and robust dipstick electrochemical sensing device, which can be built by implementation of a simple fabrication process, and which can be used in the context of a simplified method for the assessment of at least one analyte in a fluid sample.

In general, the first, second and third cavities may be directly provided within the main body.

In general, it may be preferable to provide that the removable wall is a removable foil adhered to a main face of the main body in a fluid tight manner, the removable foil being preferably made of a material which is at least partially transparent.

Advantageously, in combination or in alternative, the reference chamber may be partially delimited by a foil made of a material which is at least partially transparent and adhered to a main face of the main body in a fluid tight manner.

In general, the first and second inlet and/or outlet ports may preferably be designed so as to have two different possible states, an open state and a sealed state.

In that case, it might be advantageous to provide that the first and second inlet and/or outlet ports are preferably in their sealed state in the absence of any external action, and may be opened upon application of a pressure.

According to a particular embodiment, the measurement chamber may be provided with an additional or third inlet and/or outlet port so as to allow circulation of a fluid between the second inlet and/or outlet port and the additional inlet and/or outlet port when the removable wall is assembled to the main body.

According to another particular aspect of the present invention, each of the interfaces may preferably have a section area comprised between 10⁻⁴ mm² and 5 mm².

According to another advantageous aspect, it might be provided that at least one of the interfaces comprises a porous membrane designed in such a manner that substantially no fluid can flow through it under ambient pressure conditions, while a significant fluid flow may go through it upon application of a pressure differential, between one side of the porous membrane and the other, comprised between 0.01 bar and 5 bar. By "significant fluid flow", it should be understood that a fluid might be pushed through the bridge within a reasonable time, for instance in less than a minute.

In this case, the porous membrane may advantageously be made of at least one material taken from the group comprising a porous ceramic (alumina or glass frit), a glass wool, a porous polymer (PTFE, Nylon or Nafion), a porous carbon, and a gel-like material adapted for allowing transfer of ions, preferably including a KCI-agar solution.

In general, the sensing device of the present invention may further comprise at least one counter electrode, located in the measurement chamber, and/or at least one additional working electrode and/or at least one additional reference electrode.

The present invention further relates to a method for conducting the assessment of at least one analyte in a fluid sample, comprising steps consisting in:
a) providing a dipstick electrochemical sensing device according to the previously mentioned features, and electrically connecting its electrical connection pads to an electronic measuring unit,
b) injecting a calibration solution through the first inlet and/or outlet port and/or through the second inlet and/or outlet port until the reference chamber, the measurement chamber and the bridge are integrally filled in, the calibration solution being injected with a pressure higher than the pressure under normal temperature and pressure conditions,
c) waiting for a first predefined period of time and calibrating the sensing device with the electronic measuring unit,
d) removing the calibration solution from the measurement chamber while keeping the reference chamber and the bridge filled in with the calibration solution,
e) immersing the working electrode in a fluid sample to be assessed,
f) waiting for a second predefined period of time and assessing the analyte in the fluid sample with the electronic measuring unit.

Thanks to the method of the present invention, the sensing device is easy to handle from the fabrication site to the Point-of-Care where it will be used, as far as it might be delivered at the Point-of-Care while it still contains no fluid and thus does not need to be refrigerated. Furthermore, the calibration solution used for conducting the calibration of the sensing device can be kept as a reference stabilizing solution for the measuring operation, thus simplifying the manipulations of the sensing device and of different fluids as might be the case with prior sensing devices.

According to a preferred embodiment, step d) comprises an operation of removal of the removable wall to open the main opening of the measurement chamber.

In this case, it may be advantageous to further provide that step e) comprises an operation of dipping the sensing device in a container containing the fluid sample to be assessed, with a depth large enough for the working electrode to be fully immersed in the fluid sample.

In general, the calibration solution may preferably be chosen so as to have similar chemical properties to those of the fluid sample to be assessed, preferably a pH comprised within a predefined range of +/-2 compared to the pH of the fluid sample to be assessed, more preferably within a predefined range of +/-1.

Furthermore, when the fluid sample to be assessed is urine or saliva, the calibration solution may comprise one or several of the following:
- a buffer to keep its pH within the predefined range,
- the at least one analyte to be assessed,
- one or several salts taken in the group comprising potassium chloride, sodium chloride and calcium chloride, preferably in a range of concentrations from 0.01 mol.L⁻¹ to 3 mol.L⁻¹, and
- a dilution determinant marker, preferably in a range of concentration from 10⁻⁵ mol.L⁻¹ to 5.10⁻² mol.L⁻¹.

### Brief description of the drawings

Further details of the invention and other advantageous embodiments will appear more clearly upon reading the description below, in connection with the following figures which illustrate:
- Fig. 1: Schematic general diagram illustrating an example of a dipstick electrochemical sensing device according to a preferred embodiment of the present invention,
- Figs. 2 to 4: Schematic diagrams illustrating different steps, in chronological order, of an assessment method according to a preferred embodiment of the present invention, and
- Figs. 5 and 6: Schematic diagrams illustrating steps, in chronological order, of an assessment method according to an alternative embodiment of the present invention.

### Embodiments of the invention

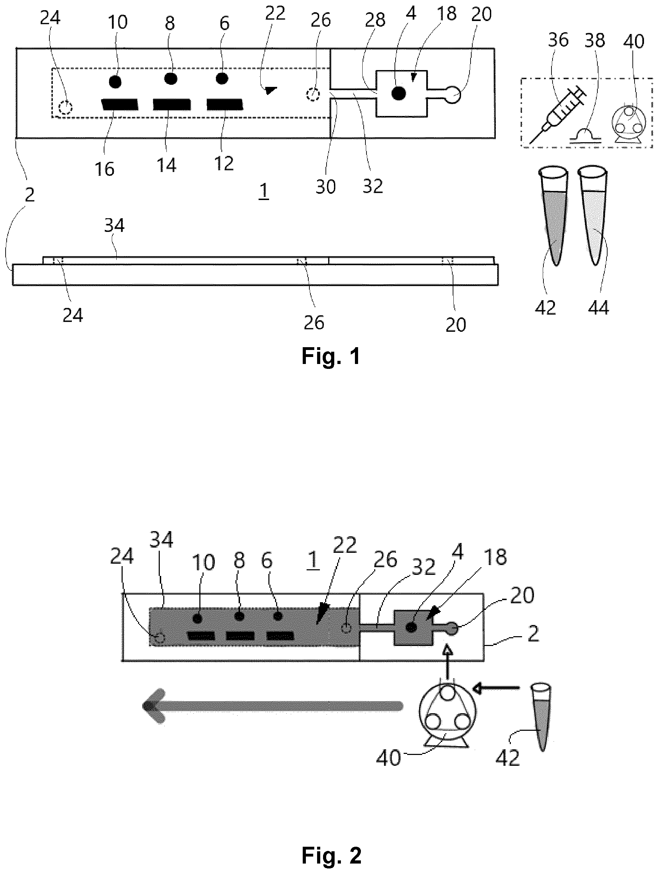

Fig. 1 is a schematic diagram illustrating a dipstick electrochemical sensing device 1 according to a preferred embodiment of the present invention, in a front view in the top part of Fig. 1 and in a lateral view in the bottom part of Fig. 1.

In general, the present invention relates to such sensing devices comprising at least one electrode, possibly an array of electrodes, which may advantageously be fabricated with the use of metallic wires and/or inks printed on different substrates such as polymers-based substrates, cellulose based substrates, or ceramic based substrate, using screen/stencil and/or inkjet printing and/or sputtering techniques.

In the illustrated embodiment, by way of a non-limiting example, the sensing device 1 comprises a substrate defining a main body 2 carrying one reference electrode 4 and three different working electrodes 6, 8 and 10 each of which being associated with an optional counter electrode 12, 14 and 16 (when the analyte of interest requires a voltametric method to be detected).

If the sensing device 1 comprises here only one shared reference electrode 4, it could of course comprise several reference electrodes without departing from the scope of the present invention as defined in the appended set of claims. Typically, the different electrodes are connected to corresponding electrical connection pads (not illustrated) to allow an electrical connection of the sensing device to a conventional electronic measuring unit in charge of converting the measures of the electrodes into analyte concentration values. Non-limiting examples of suitable electronic measuring units include Palmsens4 or Emstat pico from Palmsens, Gamry Interface 5000, uStat 400 Metrohm DropSens.

More precisely, the reference electrode 4 is located in a first cavity, defining at least part of a reference chamber 18 and provided with a first inlet and/or outlet port 20. The working electrodes 6, 8, 10 and their counter electrodes 12, 14, 16 are located in a second cavity, defining at least part of a measurement chamber 22 and provided with a second inlet and/or outlet port 24.

According to the present preferred embodiment of the sensing device 1, the measurement chamber 22 is provided with an additional or third inlet and/or outlet port 26, for reasons which will be detailed below.

The sensing device 1 is further provided with a third cavity connected to both reference and measurement chambers 18, 22 through respective corresponding interfaces 28, 30, to define a bridge 32 therebetween through which a fluid may flow.

At least the interface 30 which is located between the bridge 32 and the measurement chamber 22 has a particular structure such that it is provided with fluid flow restricting characteristics which are pressure dependent. Preferably, both interfaces 28 and 30 may have such similar properties.

These fluid flow restricting characteristics are preferably chosen so that substantially no fluid can flow through the bridge 32 under ambient pressure conditions, while a significant fluid flow may go through it upon application of a pressure differential, between one end of the bridge 32 and the other, comprised between 0.01 bar and 5 bar. As already mentioned earlier, by "significant fluid flow", it should be understood that a fluid might be pushed through the bridge 32 within a reasonable time, for instance in less than a minute.

More particularly, each of the interfaces 28, 30 may have a section area that is comprised between 10⁻⁴ mm² and 5 mm² to define a bridge 32 having properties of a micro/nanofluidic channel. Thanks to these features, capillary forces might limit the flowing aptitude of fluids through the bridge 32, unless a threshold pressure differential is applied to force a fluid to flow through the bridge 32, if necessary.

In alternative or in combination, at least one of the interfaces 28, 30 might be provided with a porous membrane (not illustrated) designed in such a manner that substantially no fluid can flow through it under ambient pressure conditions, while a significant fluid flow may go through it upon application of a pressure differential, between one side of the porous membrane and the other, comprised between 0.01 bar and 5 bar. These membranes should be electrochemically compatible with the system of interest, they should minimize the corresponding junction potentials, they should be chemically stable, and they should allow ion exchange without excessive mixing or clogging. Of course, when at least one porous membrane is provided at one end of the bridge 32, the section areas of the interfaces 28, 30 are of less importance to ensure that the adequate fluid flow restricting characteristics are fulfilled, and the one skilled in the art will encounter no particular difficulty to adapt the present disclosure to his own needs and requirements without departing from the scope of the present invention as defined in the appended claims.

Preferably, such a porous membrane may be made of at least one material taken from the group comprising:
- a porous ceramic (alumina or glass frit), which is the most commonly used material for building such membranes due to its chemical inertness, high porosity, and mechanical stability; furthermore, it provides a small but consistent liquid junction potential,
- a glass wool, which has a high porosity but can be prone to clogging over time; typically rather recommended for low cost and/or disposable sensing devices,
- a porous polymer (PTFE, Nylon or Nafion), which is particularly suitable for devices requiring long-term stability and resistance to chemical degradation; typically used in gas-sensitive or specialized electrochemical cells,
- a porous carbon, which typically have good conductive properties and inertness, and
- a gel-like material adapted for allowing transfer of ions, preferably including a KCI-agar solution; typically used for short-term and/or low cost applications as it is prone to contamination and drying out over time.

In alternative or in combination, filling membranes (not visible) might be used in the bridge 32. For instance, such filling membranes might comprise a KCI solution which is suitable for most electrochemical systems because K⁺ and Cl⁻ ions have similar mobilities, reducing junction potential. Alternative suitable solutions are CaCl₂, NaCl or LiCI solutions, or any combination of them. Alternatively, such filling membranes might comprise a AgCl-saturated KCI solution, which is more typically used when a Ag/AgCI electrode is required for stable potential and to avoid contamination by metal ions. Due to the corresponding high concentration of KCI, such a filling membrane raises a high risk of contamination in the measurement chamber 22, unless a porous barrier material is used, as mentioned earlier.

Based on what precedes, it should be understood that the bridge 32 acts like a conventional salt bridge.

Coming back to the cavities partly defining the different chambers 18, 22 and 32, they can be designed either directly in the substrate used for building the main body 2, either when fabricating the main body 2, or subsequently through an etching operation. Alternatively, the main body 2 might be substantially flat and walls might be adhered to a main face of the main body 2, after the different electrodes are built thereon, these walls not being flat to define cavities between them and the main body 2.

According to the present preferred embodiment of the sensing device 1, the measurement chamber 22 is partially delimited by a removable wall 34 which is assembled to the main body 2 in a removable manner, so that it can be removed to open a main opening through which a fluid sample can enter inside the measurement chamber 22 and contact the working electrodes 6, 8, 10 and their counter electrodes 12, 14, 16, for the purpose of conducting an assessment.

Still according to the present preferred embodiment of the sensing device 1, the reference chamber 18 and the bridge 32 are partly defined by a common wall (not visible) adhered to the main body 2, but not necessarily in a removable manner like the removable wall 34.

All the walls are adhered to the main body 2 in a fluid tight manner.

Preferably, all the walls, either removable or not, may take the form of foils comprising at least one material taken in the group comprising metals, polymers and glasses, for instance a stainless steel, aluminium, a polycarbonate, a polystyrene, a polyimide or a cyclic olefin copolymer. The walls could possibly be made of a material which is at least partially transparent so as to allow a direct visual observation of the different chambers. Thus, on top of the printed electrode substrate, a combination of a non-removable foil and a removable foil on top of the non-removable foil is assembled to form the reference chamber 18 and the measurement chamber 22. The removable foil on top of the non-removable foil can be fully or partially removed by a robotic arm, small manipulating instrument, or by the user's digits to expose the measurement chamber 22.

As far as the inlet and/or outlet ports 20, 24 and 26 are concerned, they can take any conventional form adapted for the implementation of the method according to the present invention which will be detailed below. Each of them is preferably designed so as to have two different possible states, an open state and a sealed state, while preferably being in its sealed state in the absence of any external action, and being able to be opened upon application of a pressure above a predefined level.

Different known options are available to proceed with the injection of a fluid under pressure in the sensing device 1 through one or more of its inlet and/or outlet ports 20, 24 and 26. Three different options have been schematically illustrated in Fig. 1, in a non-limiting way, in the form of a syringe 36, a blister 38 or a pump 40 (connected to a stingy or perforating member like a needle, not illustrated, to define a pneumatic delivery system for instance).

These might be implemented to inject a calibration solution 42 or a fluid sample to assess 44 inside the sensing device 1, as will be now described in connection with the diagrams of Figs. 2 to 4. Indeed, the diagrams of Figs. 2 to 4 schematically represent different steps of a method for conducting the assessment of at least one analyte in a fluid sample 44 according to a preferred embodiment of the present invention, in chronological order.

First, a sensing device 1 as was just described is provided, in a step a) and its electrical connection pads are connected to an electronic measuring unit.

In a step b), as schematically illustrated in Fig. 2, the calibration solution 42 is injected through the first inlet and/or outlet port 20 and/or through the second inlet and/or outlet port 24, until the reference chamber 18, the measurement chamber 22 and the bridge 32 are integrally filled in. During implementation of step b), the calibration solution 42 is injected with a pressure higher than the pressure under normal temperature and pressure conditions so that it can flow through the bridge 32 from one chamber to the other. Preferably, the calibration solution 42 might be injected in the first inlet and/or outlet port 20 to first fill in the reference chamber 18, before flowing through the bridge 32 and filling it in, after which it fills in the measurement chamber 22. The second inlet and/or outlet port 24 may allow a possible excess of calibration solution 42 to be expelled to avoid system over pressure.

Of course, the injection might alternatively be carried out through the second inlet and/or outlet port 24, to first fill in the measurement chamber 22, then the bridge 32 and, finally, the reference chamber 18, while any excess of calibration solution 42 could be expelled through the first inlet and/or outlet port 20.

Once all cavities are filled in, the method comprises a step c) consisting in waiting for a first predefined period of time, to reach equilibrium in the sensing device 1, before calibrating the sensing device 1 with the electronic measuring unit.

According to a preferred embodiment of the present invention, the calibration solution 42 is chosen as an "all-in-one solution", i.e. so as to have similar chemical properties to those of the fluid sample to be assessed and, at the same time, so as to be able to fulfill the function of a reference solution for the reference electrode 4 once the calibration operation is completed.

First, the calibration solution 42 may preferably have a pH comprised within a predefined range of +/-2 compared to the pH of the fluid sample 44 to be assessed, more preferably within a predefined range of +/-1, and it may comprise the analyte of interest.

By way of a non-limiting example, when the fluid sample 44 to be assessed is urine or saliva, the calibration solution 42 may preferably comprise one or several of the following:
- a buffer to keep its pH within a predefined range,
- the at least one analyte to be assessed, and
- one or several salts taken in the group comprising potassium chloride, sodium chloride and calcium chloride, preferably in a range of concentrations from 0.01 mol.L⁻¹ to 3 mol.L⁻¹.

As far as the provision of the analyte to be assessed in the calibration solution 42 is concerned, different situations might be considered. If the technology of a given sensor requires a calibration with absence of the analyte, then the calibration solution 42 should not be provided with it, of course. For instance, in the case of voltametric detection of Albumin in urine, where the functioning principle of the sensor is based on the accumulation of the analyte on the working electrode surface, the calibration is performed in the absence of Albumin, and the value measured at calibration is then subtracted from the measurement in urine. Otherwise, the analyte to be assessed might preferably be provided in the calibration solution 42 with a concentration comprised in a range of concentrations that is typical of the concentration in the specific application to be targeted and that depends on the sensor technology used. For example, if there is a threshold value for the analyte concentration that could impact a patient's therapy or put him at risk, then the concentration of the analyte in the calibration solution 42 should preferably sit near this threshold value. For example, when considering a glucose threshold concentration of 10⁻³ mol.L⁻¹, the corresponding sensor should preferably be calibrated with a calibration solution 42 including glucose with a similar concentration level. More generally, if there is no therapeutic threshold value for a given analyte of interest but the aim of an assessment is to simply have a quantitative measurement, then it could make sense to calibrate the corresponding sensor in its least precise range of detection to improve its performance precision. Alternatively, it could also make sense to calibrate the corresponding sensor in the middle of the general detection range, more particularly if the sensor does not have a well-known range in which it would be less precise than in the remainder of the general range. For example, in the case of the assessment of Na in urine, where the value can oscillate between 3.10⁻³ and 0.3 mol.L⁻¹, a calibration solution 42 including a concentration of Na comprised within this same range might preferably be used to calibrate the corresponding sensor.

Thanks to this particular choice of calibration solution 42, only one calibration step is enough to have a proper calibration of the sensing device 1, leading to a substantial simplification of the corresponding process and of the overall assessment method.

Once the calibration of the sensing device 1 is completed, an assessment of the analyte of interest in the fluid sample 44 can then be carried out.

To proceed further with the assessment, the calibration solution 42 which is present in the measurement chamber 22 needs to be replaced by the fluid sample 44, so that the latter can be in contact with the working electrodes 6, 8, 10 and their counter electrodes 12, 14, 16.

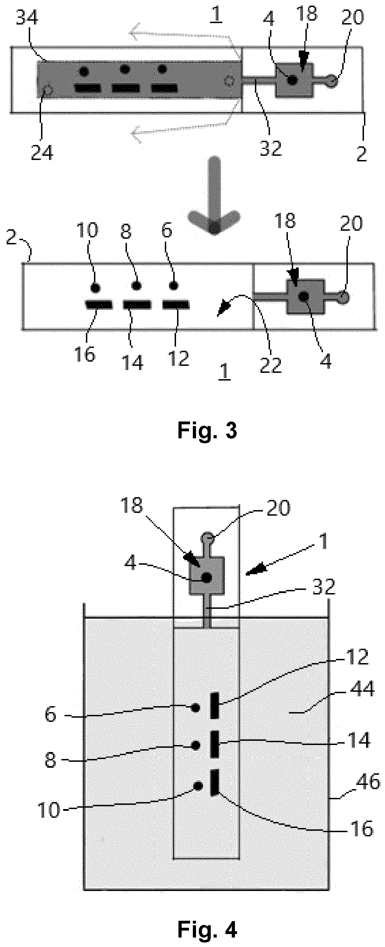

According to the preferred embodiment of the sensing device 1, the removable wall 34, or foil, can simply be removed, as schematically illustrated in Fig. 3 with arrows, to open a main opening through which the fluid sample 44 can enter inside the measurement chamber 22 and contact the working electrodes 6, 8, 10 and their counter electrodes 12, 14, 16, for the purpose of conducting the assessment.

In the case where the cavity defining the measurement chamber 22 would be arranged in the main body 2, the fluid sample 44 could be poured directly into the cavity to have all the working electrodes 6, 8, 10 and their counter electrodes 12, 14, 16 immersed in the fluid sample 44.

Alternatively, as illustrated in Fig. 4, the sensing device 1 may preferably be used as dipstick device and be directly dipped in a container 46 containing the fluid sample 44 to be assessed, with a depth large enough for the working electrodes 6, 8, 10 and their counter electrodes 12, 14, 16 to be fully immersed in the fluid sample 44.

It is noticeable that, thanks to the specific choice for the composition of the calibration solution 42, more particularly to the fact that the calibration solution 42 includes the necessary species to fulfill the function of a reference solution or electrolyte for the reference electrode 4, the calibration solution 42 can be kept inside the reference chamber 18 and inside the bridge 32 during the assessment step. The fact that one and same solution can be successively used as a calibration solution and, later, as a reference solution also leads to an important simplification of the assessment method.

Furthermore, it should also be noted that a mixing phenomenon is taking place during the measuring step, between the calibration/reference solution 42 and the fluid sample 44. This mixing phenomenon is essentially a combination of the circulation of fluids through the bridge 32, and a diffusion of the highly concentrated buffer entity out of the calibration/reference solution 42 and might lead to a possible modification of the surrounding medium of the reference electrode 4, which may offset the reference potential of the reference electrode 4. This mixing phenomenon depends on the fluids in play but also on the transversal dimensions of the chambers/channels. The one skilled in the art will not encounter any particular difficulty in adjusting the length of the bridge 32 as a function of the relevant parameters. For instance, when using a KCI buffer with a bridge 32 having a diameter of 2 mm, the circulation of fluids might extend approximately up to 2 mm inside the bridge 32 from the measurement chamber 22, while the diffusion of KCI might extend up to 3 mm during an assessment of 20 min which is already on the high side of the range regarding mean assessment times. Indeed, the equation x≈2(Dt)^{1/2} gives a general estimate of the diffusion length x over time t in an ideal, unbounded medium, with D being the diffusion coefficient of KCI (of the order of 1.844.10⁻⁹ m².s⁻¹ at 25°C in an aqueous medium). So KCl would diffuse by 1.5 mm after 5 min, and by 3.0 mm after 20 min.

When dealing with a confined space like a channel, the physical dimensions (WxLxH) could affect diffusion depending on the degree of confinement and the interaction with the walls and slow down the diffusion. So, the situation in a confined channel is very safe with a length of 3 mm. But we need to also consider the circulation of the solution previously mentioned, which we kept larger than what it really is by assuming 2mm. Thus, the minimum length for the bridge 32 should be 3 mm + 2 mm = 5 mm.

Finally, the assessment method comprises another step f) consisting in waiting for a second predefined period of time and assessing the analyte in the fluid sample 44 with the electronic measuring unit.

According to an alternative embodiment of the assessment method, the measurement chamber 22 can be kept closed for the assessment steps b) and c), i.e. the removable wall 34 would be kept in its initial configuration, still assembled to the main body 2. The calibration solution 42 could then be pushed outside the measurement chamber 22 by the injection under pressure of the fluid sample 44, preferably through the additional or third inlet and/or outlet port 26, or possibly through the second inlet and/or outlet port 24. Of course, then the calibration solution 42 would be ejected out of the measurement chamber 22 through the other port of the chamber which is not used to inject the fluid sample 44.

This approach might be particularly advantageous if the available volume of fluid sample is not enough to dip the sensing device 1 therein for carrying out the measurement operation.

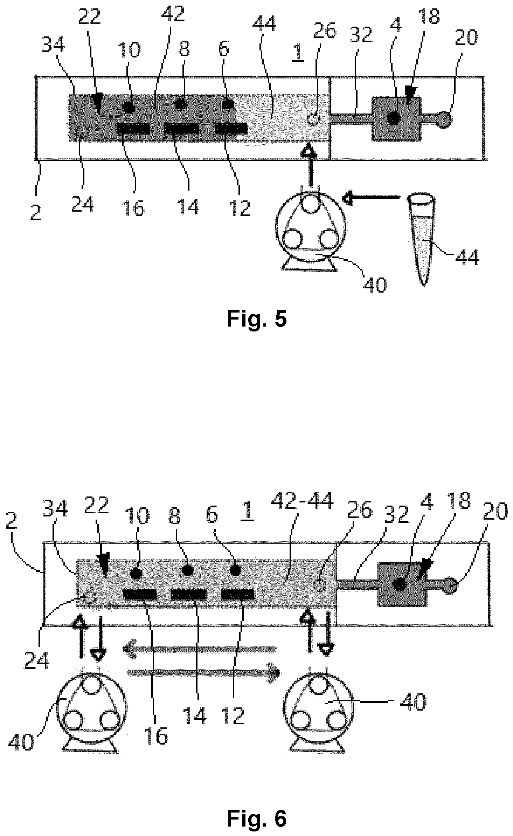

Such an approach, when the removable wall 34 is kept assembled to the main body 2, might be further used as a basis for an alternative assessment method of the present invention, as schematically illustrated in Figs. 5 and 6, in chronological order.

Indeed, in this case, an exact amount of fluid sample 44 can be injected into the measurement chamber 22, through the second inlet and/or outlet port 24 or through the third inlet and/or outlet port 26. As previously described, the injection of the fluid sample 44 through one port of the measurement chamber 22 pushes the calibration solution 42 outside the measurement chamber 22 through the other port of the measurement chamber 22, without impact on the reference chamber 18 which remains filled in with the calibration(/reference) solution 42.

As long as the volume of fluid sample 44 which is injected inside the measurement chamber 22 is smaller than the overall volume of this chamber 22, it results in the dilution of the fluid sample 44 in/by the calibration solution 42.

Fig. 5 schematically illustrates an alternative step following the end of the calibration operation as previously described, i.e. the injection of the fluid sample 44 inside the measurement chamber 22 through the third inlet and/or outlet port 26, while the calibration solution 42 is ejected outside of the measurement chamber 22 through the second inlet and/or outlet port 24.

Once the desired amount of fluid sample 44 was injected into the measurement chamber 22, a mixing of the fluid sample 44 and of the remaining calibration solution 42 may preferably be implemented, to obtain the situation as schematically illustrated in Fig. 6 of a homogeneous solution.

The mixing operation may be implemented thanks to the use, for instance, of a syringe pump system connected to both inlet and/or outlet ports 24, 26, and/or at least one of a peristaltic pump system, a diaphragm pump system, a gear pump system, a metering pump system, a microfluidic valve and pump system, and a pneumatic delivery system.

Once the mixing is completed, an assessment operation as mentioned above can be carried out while the removable wall 34 is still assembled to the main body 2.

It should be noted that, in the case of the implementation of a dilution of the fluid sample 44 inside the sensing device 1, the calibration solution 42 may advantageously further comprise a dilution determinant marker, preferably in a range of concentration from 10⁻⁵ mol.L⁻¹ to 5.10⁻² mol.L⁻¹, to allow the checking of the exact dilution ratio between the calibration solution and the fluid sample, more especially when a precise sample dilution is needed. The dilution determinant marker may be taken in the group comprising ferricyanide, ferrocene, ruthenium hexamine, methylene blue and similar. If necessary, such a dilution determinant marker may be desiccated or freeze-dried onto the working electrode(s) 6, 8, 10 or placed in a designated location in the measurement chamber 22, and subsequently dissolved when the calibration solution 42 is directed through the measurement chamber 22.

It appears from what precedes that the present invention relates to a sensing device comprising at least one screen-printed electrode that can be fabricated using either screen printing, inkjet printing, or sputtering. This electrode comprises a conductive ink formulation for screen and inkjet printing processes, and a target material for sputtering, tailored to enhance the electrode's electrical, mechanical, and surface properties. The invention also includes a substrate compatible with all three manufacturing processes, ensuring the electrode's broad applicability. The substrate is designed to be versatile and compatible with the conductive layers formed through screen printing, inkjet printing, or sputtering. It can be fabricated from a range of materials, including ceramic, polymer, or glass, depending on the specific requirements of the desired application. Alternatively, the electrodes could be fabricated by using metallic wires, as previously mentioned.

The calibration solution can be prepared using any suitable buffer to maintain a stable pH environment, thus ensuring the reliability and reproducibility of the electrode's measurements across a wide range of conditions. The calibration solution preferably further contains a fixed concentration of the analyte, preferably chosen to match the expected range of detection in the application of interest. This standardized approach to calibration allows for the precise tuning of the electrode's response, facilitating its application in diverse analytical settings. The choice of buffer and analyte concentration can be tailored to the specific requirements of the intended application, offering further customization and flexibility in the electrode's use. If necessary, a required amount of analyte can be desiccated or freeze-dried onto the working electrode(s) or placed in a designated location in the measurement chamber, and subsequently dissolved when the calibration solution is directed through the measurement chamber. Furthermore, the calibration solution is preferably formulated to include an optimal concentration of chloride salt. This addition is particularly relevant for stabilizing the reference electrode. The presence of chloride ions is important for maintaining the electrochemical equilibrium at the reference electrode interface, thereby enhancing the accuracy and reliability of the electrode system as a whole. This tailored approach to the calibration solution composition not only facilitates precise analyte detection but also contributes to the system's overall stability and efficiency, especially in long-term and continuous monitoring applications. By allowing for the customization of the buffer, analyte concentration, and chloride salt levels, this solution underscores the invention's adaptability to a wide array of analytical challenges.

The sensing device according to the invention can be customized to detect a single analyte by incorporating only one sensor, or several sensors can be added to enable multiplexed/simultaneous detection of numerous analytes, hence enhancing the diagnostic capabilities by providing more detailed information. This comprehensive method is invaluable for monitoring illnesses such as diabetes and its associated consequences.

For example, other analytes that could be monitored alongside glucose in urine are:
- pH: Abnormal pH levels can indicate various conditions, including urinary tract infections or metabolic issues,
- Electrolytes (e.g., sodium, potassium): these can provide information about kidney function and overall metabolic status,
- Proteins (Albumin): protein in the urine can indicate kidney damage, which is a common complication of diabetes, and
- Creatinine: can be used to assess kidney function and calculate the albumin-to-creatinine ratio, which helps detect early kidney damage.

For example, other analytes that could be monitored alongside glucose in saliva are:
- Cortisol: stress hormone levels can be measured to assess stress and adrenal function,
- Electrolytes (e.g., sodium, potassium): important for assessing overall electrolyte balance,
- pH: saliva pH can indicate oral health status and potential metabolic conditions, and
- Proteins (C-Reactive Protein (CRP)): it is a marker of inflammation, and its levels can provide valuable insights into the body's inflammatory status.

The invention described herein presents several advantages in comparison to the prior existing solutions, including:
- A matrix independent calibration system using a singular solution for calibration,
- A microfluidic chip with two separate chambers (reference and measurement) connected by a micro/nanofluidic channel that acts as a salt bridge,
- A system of syringes/pumps to simultaneously inject a fluid sample, while protecting the reference electrode from the fluid sample, and calibrate the testing sensor(s),
- An electrochemical sensing element(s) embedded in a chamber through a removable foil,
- A detachable foil that, when removed, enables direct measurement in the solution under test using a dipstick configuration, while ensuring that the reference electrode is safeguarded from the same solution,
- One or more electrochemical sensing elements sharing a same reference electrode,
- A dilution mixing feature which allows to dilute, when needed, complex matrixes and precisely controlling the dilution ratio.

The one skilled in the art will encounter no particular difficulty to adjust the present teaching to his specific needs and/or requirements, without departing from the scope of the present invention as defined by the appended set of claims.

## Claims

1. Dipstick electrochemical sensing device (1), for assessing at least one analyte in a fluid sample (44), comprising a main body (2) provided with at least one reference electrode (4) and at least one working electrode (6, 8, 10), said reference and working electrodes (4, 6, 8, 10) being connected to corresponding electrical connection pads,
wherein said reference electrode (4) is located in a first cavity, defining at least part of a reference chamber (18) and provided with a first inlet and/or outlet port (20), while said working electrode (6, 8, 10) is located in a second cavity, defining at least part of a measurement chamber (22) and provided with a second inlet and/or outlet port (24),
wherein the sensing device (1) is further provided with a third cavity connected to both reference and measurement chambers (18, 22) through corresponding interfaces (28, 30), to define a bridge (32) therebetween through which a fluid may flow, at least the interface (30) located between said bridge (32) and said measurement chamber (22) having such a structure that it is provided with fluid flow restricting characteristics which are pressure dependent, and
wherein said measurement chamber (22) is partially delimited by a removable wall (34) which is assembled to said main body (2) in a removable manner, so that it can be removed to open a main opening through which the fluid sample can enter inside said measurement chamber (22) and contact said at least one working electrode (6, 8, 10) for the purpose of conducting an assessment.

2. Sensing device (1) according to claim 1, wherein said first, second and third cavities are provided within said main body (2).

3. Sensing device (1) according to claim 1 or 2, wherein said removable wall (34) is a removable foil adhered to a main face of said main body (2) in a fluid tight manner, said removable foil being preferably made of a material which is at least partially transparent.

4. Sensing device (1) according to any of the preceding claims, wherein said reference chamber (18) is partially delimited by a foil made of a material which is at least partially transparent and adhered to a main face of said main body (2) in a fluid tight manner.

5. Sensing device (1) according to any of the preceding claims, wherein said first and second inlet and/or outlet ports (20, 24) are designed so as to have two different possible states, an open state and a sealed state.

6. Sensing device (1) according to claim 5, wherein said first and second inlet and/or outlet ports (20, 24) are preferably in their sealed state in the absence of any external action, and may be opened upon application of a pressure above a predefined level.

7. Sensing device (1) according to any of the preceding claims, wherein said measurement chamber (22) is provided with an additional inlet and/or outlet port (26) so as to allow circulation of a fluid between said second inlet and/or outlet port (24) and said additional inlet and/or outlet port (26) when said removable wall (34) is assembled to said main body (2).

8. Sensing device (1) according to any of the preceding claims, wherein each of said interfaces (28, 30) has a section area comprised between 10⁻⁴ mm² and 5 mm².

9. Sensing device (1) according to any of the preceding claims, wherein at least one of said interfaces (28, 30) is provided with a porous membrane designed in such a manner that substantially no fluid can flow through it under ambient pressure conditions, while a significant fluid flow may go through it upon application of a pressure differential, between one side of the porous membrane and the other, comprised between 0.01 bar and 5 bar.

10. Sensing device (1) according to claim 9, wherein said porous membrane is made of at least one material taken from the group comprising a porous ceramic (alumina or glass frit), a glass wool, a porous polymer (PTFE, Nylon or Nafion), a porous carbon, and a gel-like material adapted for allowing transfer of ions, preferably including a KCI-agar solution.

11. Sensing device (1) according to any of the preceding claims, further comprising at least one counter electrode (12, 14, 16), enclosed in said measurement chamber (22), and/or at least one additional working electrode (6, 8, 10) and/or at least one additional reference electrode.

12. Method for conducting the assessment of at least one analyte in a fluid sample (44), comprising steps consisting in:
a) providing a dipstick electrochemical sensing device (1) according to any of claims 1 to 11 and electrically connecting its electrical connection pads to an electronic measuring unit,
b) injecting a calibration solution (42) through said first inlet and/or outlet port (20) and/or through said second inlet and/or outlet port (24) until said reference chamber (18), said measurement chamber (22) and said bridge (32) are integrally filled in, said calibration solution (42) being injected with a pressure higher than the pressure under normal temperature and pressure conditions,
c) waiting for a first predefined period of time and calibrating the sensing device (1) with said electronic measuring unit,
d) removing said calibration solution (42) from said measurement chamber (22) while keeping said reference chamber (18) and said bridge (32) filled in with said calibration solution (42),
e) immersing said working electrode (6, 8, 10) in a fluid sample (44) to be assessed,
f) waiting for a second predefined period of time and assessing the analyte in the fluid sample (44) with the electronic measuring unit.

13. Method according to claim 12, wherein step d) comprises an operation of removal of said removable wall (34) to open said main opening of said measurement chamber (22).

14. Method according to claim 13, wherein step e) comprises an operation of dipping said sensing device (1) in a container (46) containing the fluid sample (44) to be assessed, with a depth large enough for said working electrode (6, 8, 10) to be fully immersed in the fluid sample (44).

15. Method according to any of claims 12 to 14, wherein said calibration solution (42) is chosen so as to have similar chemical properties to those of the fluid sample (44) to be assessed, preferably a pH comprised within a predefined range of +/-2 compared to the pH of the fluid sample (44) to be assessed, more preferably within a predefined range of +/-1.

16. Method according to claim 15, when the fluid sample (44) to be assessed is urine or saliva, wherein said calibration solution (42) comprises one or several of the following:
- a buffer to keep its pH within said predefined range,
- the at least one analyte to be assessed,
- one or several salts taken in the group comprising potassium chloride, sodium chloride and calcium chloride, preferably in a range of concentrations from 0.01 mol.L⁻¹ to 3 mol.L⁻¹, and
- a dilution determinant marker, preferably in a range of concentration from 10⁻⁵ mol.L⁻¹ to 5.10⁻² mol.L⁻¹.
